# EUROPÄISCHE PATENTANMELDUNG

(11) **EP 2 957 758 A1**
(43) Veröffentlichungstag der Anmeldung: **23.12.2015**
(21) Anmeldenummer: 15164792.2
(22) Anmeldetag: 23.04.2015
(51) Int. Cl.: F02M 37/22, B01D 36/00, B60R 13/08, F16L 59/00, G01N 33/28, H02G 3/04

(54) **Sensoreinrichtung, Filteranordnung und Verfahren zum herstellen derselben**

(30) Priorität: 20.06.2014 DE 102014008830
(71) Anmelder: MANN + HUMMEL GMBH, 71638 Ludwigsburg (DE)
(72) Erfinder: ANDRES, Thomas, 67240 Bobenheim/Roxheim (DE)

(57) **Zusammenfassung**

Eine Sensoreinrichtung (1) für eine Filteranordnung (100) umfasst mindestens ein Sensorelement (2) zum Erfassen eines Betriebsparameters für die Filteranordnung (100), insbesondere zum Detektieren von Wasser, und ein Brandschutzmaterial (11, 25), welches das Sensorelement (2) zumindest teilweise umgibt.

## Beschreibung

### Technisches Gebiet

Die vorliegende Erfindung betrifft eine Sensoreinrichtung für eine Filteranordnung, wie für ein Betriebsmittelfilter in einem Fahrzeug. Ferner betrifft die Erfindung ein Verfahren zum Herstellen einer derartigen Sensoreinrichtung.

In bestimmten Filteranwendungen ist die automatische Erfassung der voneinander getrennten Stoffe gewünscht. Beispielsweise werden bei Kraftstofffiltern, wie aus der WO2012/025405A1 bekannt, insbesondere für Dieseleinspritzmotoren, Wasserbestandteile aus dem Dieselkraftstoff abgeschieden bzw. gefiltert. Das abgeschiedene Wasser wird in der Regel aufgefangen und dessen Füllstand mit Hilfe eines Feuchtigkeitssensors oder Wassersensors erkannt. Eine zuverlässige Funktion der Filteranordnung und/oder des entsprechenden Sensors auch unter besonderen Betriebsbedingungen ist dabei gewünscht. Die Sensoren oder Sensoreinrichtungen sind in der Regel getrennt von dem eigentlichen Filterelement vorgesehen. Beispielsweise kann ein Sensor mit einem in den Innenraum des Filterelements reichenden Messfühler mit dem Filterelement fluiddicht verbunden werden, so dass während des Betriebs Betriebsparameter des Filterelements erfasst werden können. Die eigentliche Sensoreinrichtung ist dabei außerhalb des Filterelementes angeordnet, beispielsweise angeschraubt, angeflanscht oder gesteckt.

### Offenbarung der Erfindung

Vor diesem Hintergrund liegt der vorliegenden Erfindung die Aufgabe zugrunde, eine verbesserte Sensoreinrichtung zu schaffen.

Demgemäß wird eine Sensoreinrichtung für eine Filteranordnung vorgeschlagen, welche mindestens ein Sensorelement zum Erfassen eines Betriebsparameters für die Filteranordnung und ein Brandschutzmaterial, welches das Sensorelement zumindest teilweise umgibt, aufweist.

Das Brandschutzmaterial schützt zum Beispiel das Sensorelement vor hohen Temperaturen, die eine Funktionsweise des Sensorelementes oder eine Ankopplung des Sensorelements mit der Filteranordnung beeinträchtigen können. In Ausführungsformen umgibt das Brandschutzmaterial das Sensorelement vollständig oder zu großen Teilen. Das Brandschutzmaterial kann auch so geformt sein, dass es einen Aufnahmebereich für das Sensorelement umfasst.

Ein Brandschutzmaterial schirmt dabei von außen auf die Filteranordnung einwirkende Flammen oder Hitze in Folge eines Brandes auf das Sensorelement derart ab, dass bei einem vorgegebenen Zeitraum und bei vorgegebenen Flammtemperaturen die Funktion des Sensorelements und/oder eine Abdichtung des Sensorelements mit dem eingesetzten Filter unbeeinträchtigt bleibt. Ein Betriebsparameter ist beispielsweise das Vorliegen von Wasser in oder an der Filteranordnung. Denkbar ist jedoch auch die Ausgestaltung des Sensorelements zum Erfassen von Fluiden, wie Gasen oder Flüssigkeiten, Temperaturen, Drücken, elektrischen Leitwerten oder dgl.. Das Sensorelement ist insbesondere ein Wassersensor.

In Ausführungsformen eignet sich die Sensoreinrichtung insbesondere für eine Filteranordnung, die zum Filtern von Betriebsmitteln in Fahrzeugen, wie Kraftfahrzeugen, Wasserfahrzeugen, Schienenfahrzeugen. stationären Stromerzeugern oder Fluggeräten, eingerichtet sind. Die Sensoreinrichtung ist insbesondere in oder an einem Filter wie einem Kraftstofffilter, einem Diesel-Kraftstofffilter für Diesel-Einspritzmotoren, angebracht. Man kann auch von Preline- oder Vorfiltern sprechen, die als Wechselfilter zur Partikel- und Wasserabscheidung von Kraftstoffen ausgestaltet sind. Als Wechselfilter werden im Ganzen austauschbar an einen Filterkopf montierbare Filter mit einem geschlossenen, meist aus Metall ausgeführten Gehäuse und einem darin angeordneten Filterkörper bezeichnet. Diese Filter sind auch als Spin-On Filter bekannt, da sie meist mittels eines Gewindes an den Filterkopf montierbar sind.

In Ausführungsformen umfasst das Brandschutzmaterial ein intumeszierendes Material. Intumeszierende Materialien expandieren bei thermischer Beanspruchung bzw. schäumen auf und bilden eine dicke wärme- und flammenisolierende Schutzschicht. Intumeszierende Materialien sind auch als Brandschutzschäume bekannt. Als Brandschutzmaterial kommt insbesondere ein Polyurethanschaum in Frage, welcher mit Brandschutzadditiven versetzt ist. Das Brandschutzmaterial kann als Formteil in einer geeigneten Geometrie in der Sensoreinrichtung vorliegen. Es ist auch möglich, dass das Sensorelement mit entsprechendem Schaummaterial umschäumt ist. Bevorzugt weist das intumeszierende Material eine Dichte von kleiner 1000 kg/m³, besonders bevorzugt kleiner 400 kg/m³ auf.

In weiteren Ausführungsformen umfasst das Brandschutzmaterial ein flammbeständiges Faser-material. Es sind beispielsweise Glasfilamentgewebe bekannt, die als Hitzeschilde verwendet werden können. Das Brandschutzmaterial kann zum Beispiel Silikatglasfasern umfassen, die Filamentdurchmesser von > 6 µm haben. Insbesondere Mineral- oder Glasfasermaterialien sind temperaturbeständig, in der Regel bis zu 1.000°C, und haben gute Isoliereigenschaften. Insbesondere Nadelmatten als Brandschutzmaterialien sind nicht brennbar und haben eine gute Vibrationsbeständigkeit. Ein Fasermaterial umfassendes Brandschutzmaterial kann als Formteil um das jeweilige Sensorelement gefertigt sein. Es ist möglich, das Brandschutzmaterial mit Befestigungsmitteln, wie Schellen, Gehäuseteilen, Netzen oder anderen ein räumliches Fixieren ermöglichenden Mitteln an oder um das Sensorelement zu befestigen.

Das Brandschutzmaterial kann ferner ein Keramikmaterial aufweisen. Keramik kann beispielsweise als Vergussmasse eingesetzt werden und das Sensorelement zumindest teilweise umschließen. Das Keramikmaterial weist insbesondere eine Aluminium- und/ oder Kalziumverbindung auf. Als Keramikmaterialien kommen in Frage: Korund mit Kalziumaluminatbindung, Aluminiumoxid, Kalziumoxid, Magnesiumoxid, Eisenoxid sowie Mischungen der vorgenannten Materialien.

In einer Ausführungsform ist das Brandschutzmaterial ein Edelstahlmaterial, welches das Gehäuse des Sensorelementes bildet und dieses vollständig umgibt. Auf das Gehäuse einwirkende Wärme kann teilweise über das Sensorelement an das im Inneren des Filters und des Sensors befindliche Wasser abgeführt werden, was den Aufwärmvorgang im Brandfalle verzögert. Das Gehäuse ist dabei bevorzugt im an den Filter angrenzenden Bereich mit einer ringscheibenförmigen Auskragung versehen, die radial deutlich (d.h. mindestens 5 mm und bevorzugt mindestens 10 mm) über den Anschlussbereich des Sensorelementes mit Anschlussgewinde und Dichtung hinausragt. Auf diese Weise können Flammen abgelenkt werden, so dass die Gefahr eines direkten Kontaktes des Anschlussbereiches mit Flammen deutlich verringert wird.

In Ausführungsformen ist an das Brandschutzmaterial wenigstens eine Deckplatte angebracht, Beispielsweise kann das Brandschutzmaterial zwischen zwei Deckplatten angeordnet sein. Als Deckplattenmaterial kann ein Kohlenstoffmaterial eingesetzt werden. Es kommen insbesondere Carbonfaser- oder Polyacrylnitril-(PAN)-Fasermaterialien als Deckplattenmaterial in Frage. Die Dicke der Deckplatte ist beispielsweise zwischen 1 und 3 mm. Blähgraphit schäumt zum Beispiel bereits ab 145°C auf und expandiert. Graphit hat insbesondere den Vorteil, dass es alterungsbeständig, wasserbeständig und UV-beständig ist.

In weiteren Ausführungsformen umfasst das Brandschutzmaterial und/oder das Deckplattenmaterial ein Blähgraphit.

In Ausführungsformen ist ein zylinderförmiges Brandschutzmaterial zwischen zwei Deckplatten, die die Deckflächen bilden vorgesehen.

In Ausführungsbeispielen ist das Sensorelement von einem umschließenden Brandschutzgehäuse umgeben. Das Brandschutzgehäuse ist zur Aufnahme des Brandschutzmaterials um das Sensorelement herum ausgestaltet. Beispielsweise kann das Sensorelement einerseits an die Filteranordnung oder einen Filter grenzen und andererseits von einem Brandschutzgehäuse umgeben sein, welches im Falle einer Flammeneinwirkung von außen Schutz bietet. Das Brandschutzgehäuse kann aus einem Metallwerkstoff gefertigt sein. Das Brandschutzgehäuse ist zum Beispiel mit einem Handhabungsabschnitt ausgestattet, um eine Einheit aus dem Sensorelement, dem Brandschutzmaterial und dem Gehäuse von dem Filter der Filteranordnung zu trennen.

In Ausführungsformen hat das Gehäuse Öffnungen. Durch die Öffnungen kann beispielsweise im Brandfall aufschäumendes Material unter einer Volumenvergrößerung heraustreten und somit das Sensorelement besser schützen. Es ist auch möglich, dass innerhalb des Brandschutzgehäuses das Sensorelement und das Brandschutzmaterial derart miteinander verbunden sind, dass eine zerstörungsfreie Lösbarkeit der Teile voneinander unmöglich ist.

In Ausführungsformen der Sensoreinrichtung ist zwischen dem Brandschutzmaterial und dem Sensorelement ein Hohlraum vorgesehen. Dieser Hohlraum oder ein Spalt zwischen der äußeren Oberfläche des Sensorelements und dem Brandschutzmaterial führt zu einer verbesserten Isolation nach außen hin.

Die Sensoreinrichtung kann in Ausführungsformen ein Sensorelement umfassen, bei dem mindestens eine Anschlussleitung zum Übermitteln von Sensorsignalen vorgesehen ist. Die Anschlussleitung ist dabei wenigstens teilweise durch das Brandschutzmaterial nach außen geführt. Die Anschlussleitung ist beispielsweise ein Metalldraht, der mit einer Kunststoffisolation versehen ist. Die Anschlussleitung ist insbesondere ein Kabel in flammgeschützter Ausführung. Vorzugsweise entspricht das Kabel DIN 5510 mit Brandschutzstufe 1, 2, 3 oder 4.

Die Sensoreinrichtung ist in Ausführungsformen derart mit einem Brandschutzmaterial ausgestattet, dass das Sensorelement einer Beflammung der Sensoreinrichtung über einen vorgegebenen Zeitraum bei einer vorgegebenen Temperatur widersteht. Denkbar ist beispielsweise, dass der vorgegebene Zeitraum mindestens 20, vorzugsweise mindestens 30 Minuten und noch bevorzugter 40 Minuten beträgt. Die Flammtemperatur ist beispielsweise mindestens 700°C, vorzugsweise 800°C und weiter bevorzugt 900°C. Es ist insbesondere denkbar, dass die Sensoreinrichtung eingerichtet ist, eine Feuerwiderstandsprüfung nach ISO 7840, ISO 19 921 oder nach Vorschriften der Schifffahrts-Klassifikationsgesellschaften zu bestehen.

Die Sensoreinrichtung für die Filteranordnung ist dabei insbesondere derart ausgestaltet, dass an einer Verbindung zwischen dem Sensorelement und der Filteranordnung, wie beispielsweise einem Filter oder Wechselfilter, keine Undichtigkeit auftritt. Eine Kopplung zwischen der Filteranordnung und dem Sensorelement ist vorzugsweise derart fluiddicht, dass bei Druckunterschieden im einstelligen bar-Bereich keine Leckage auftritt.

Es wird ferner eine Filteranordnung mit einem Filter, insbesondere einem Wechselfilter, zum Filtern eines Fluids mit einer, wie vorbeschriebenen Sensoreinrichtung vorgeschlagen. Das Sensorelement ist dabei fluiddicht an dem Filter angebracht.

Vorzugsweise ist eine Dichtungseinrichtung, beispielsweise in der Art eines Dichtungsrings, zwischen dem Sensorelement und dem Filter vorgesehen.

Die Filteranordnung ist insbesondere zum Einsatz in Wasser- und Schienenfahrzeugen und zum Filtern von Kraftstoff für einen Verbrennungsmotor ausgestaltet. Das Sensorelement dient dabei dem Detektieren von Wasser in einem Aufnahmeraum des Filters oder der Filteranordnung.

Die Filteranordnung kann in Ausführungsformen eine Halteeinrichtung für den Filter, das Brandschutzgehäuse und/oder eines expandierenden Brandschutzmittels umfassen. Als Halteeinrichtung kommen insbesondere Schellen, Stege, Wülste, Rinnen, Öffnungen oder Hinterschnitte in Frage. Beispielsweise kann ein Brandschutzgehäuse für das Sensorelement mit Hilfe einer Schelle und einem oder mehreren Haltelaschen am Filter und/oder am Filterkopf befestigt sein.

Schließlich wird ein Verfahren zum Herstellen einer Sensoreinrichtung, wie zuvor beschrieben, vorgeschlagen. Dabei wird ein Sensorelement mit einem (zunächst) fluiden Brandschutzmaterial wenigstens teilweise ummantelt. Das Brandschutzmaterial ist in einem abgebundenen verfestigten Zustand intumeszierend.

Das Verfahren eignet sich insbesondere zum einfachen Herstellen von Sensoreinrichtungen, die Anforderungen an feuerwiderstandsfähige Systeme, insbesondere in Wasser- und Schienenfahrzeugen, genügen. In Ausführungsformen wird beispielsweise das Sensorelement mit einem Brandschutzschaum umgossen oder umspritzt. Denkbar ist das Bereithalten eines becherförmigen Brandschutzgehäuses, welches mit nicht abgebundenem Brandschutzmaterial verfüllt ist und das Sensorelement hineingesteckt wird. Ferner ist denkbar, eine Gießform bereitzuhalten, den Sensor in der Form zu positionieren und anschließend Brandschutzschaummasse in die Gießform einzudosieren, der beim Aufschäumen das Sensorelement sowie besonders bevorzugt auch den an das Sensorelement angrenzenden Bereich eines Anschlusskabels und eines Wasserablaufrohrs für im Filter abgeschiedenes Wasser umschließt und sich mit diesen verbindet. Bevorzugt ist der Brandschutzschaum nach dem Aushärten derart drehmomentfest (für bei Handbedienung üblicherweise auftretende Momente) mit dem Sensorelement verbunden, dass das Sensorelement durch Drehung am Brandschutzschaum in ein dafür am (Wechsel-)filter vorgesehenes Gewinde einschraubbar ist. Dazu können am Sensorelement außen bevorzugt Formschlusselemente vorgesehen sein. Beispielsweise kann das Sensorelement an seiner Außenseite (radial außen in Bezug auf die Mittelachse) eine polygonale Form aufweisen oder mit Rippen, Rändeln oder sonstigen zur Drehmomentübertragung geeigneten Strukturen versehen sein, die vom Brandschutzschaum formschlüssig umgeben sind. Bevorzugt ragt das Wasserablaufrohr räumlich unten aus dem Brandschutzschaum heraus. Dies erleichtert sowohl Bedienung als auch Herstellung.

In Ausführungsformen der Filteranordnung hat der Filter einen Einlass und einen Auslass und dient dem Filtern von Betriebsmitteln für Verbrennungsmotoren. Das der Filter kann eine zylinderförmige Form haben und in bestimmungsgemäßem Einbau an seiner Unterseite eine Wasserablassöffnung sowie einen Anschluss für ein Sensorelement.

Ein Gehäuse des Wechselfilters, bzw. des Filters ist beispielsweise aus feuerfestem Material mit einem Schmelzpunkt größer 900°C, wie einem Metallwerkstoff, gefertigt. Darüber hinaus kann die Filteranordnung beispielsweise in der Art eines Vorfiltersystems mit einem Filterkopf, der optional eine Pumpe, einen Heizelement und Schlauchanschlüsse für Ein- und Auslass umfasst, ausgeführt sein. Die Filteranordnung erfüllt insbesondere eine Vorfilterfunktion mit einer Wasserabscheidung. Die Filteranordnung wird mit einem Betriebsdruck von -1 bis 7 bar oder 3 bis 5 bar betrieben. Dementsprechend sind fluiddichte Übergänge zwischen Filter, Filterkopf und dem angeschlossenen Sensorelement für Über- oder Unterdrücke bis 8 bar ausgelegt.

Ein entsprechender Filter ist mit einem Filterkörper aus einem Filtermedium ausgestattet. Das Filtermedium kann ein flächiges Material sein, das gefaltet oder wellenförmig ausgebildet ist. Als Faltungen sind beispielsweise Zickzack- oder W-Faltungen bekannt. Das Filtermedium kann geprägt und anschließend an Prägekanten unter Ausbildung von Faltkanten scharfkantig gefaltet sein. Als Ausgangsmaterial kann ein flächiger Filtermaterialbogen dienen, welcher entsprechend umgeformt wird. Es kann ein Endlosfaltenbalg ausgebildet sein.

Das Filtermedium ist beispielsweise ein Filtergewebe, ein Filtergelege oder ein Filtervlies. Insbesondere kann das Filtermedium in einem Spinnvlies- oder Meltblown-Verfahren hergestellt sein. Weiter kann das Filtermedium verfilzt oder genadelt sein. Das Filtermedium kann Naturfasern, wie Baumwolle, oder Kunstfasern, beispielsweise aus Polyester, Polyvinylsulfit oder Polytetrafluorethylen, aufweisen. Die Fasern können bei der Verarbeitung in, schräg und/oder quer zur Maschinenrichtung orientiert sein. Das Filtermedium kann ein- oder mehrlagig sein. Das zu filternde Medium ist beispielsweise Luft. Ein gasförmiges Medium bzw. Luft umfasst hier auch Gas- bzw. Luftfeststoffgemische und/oder Gas- bzw. Luft-Flüssigkeitsgemische. Genauso sind Betriebsflüssigkeiten, wie Kraftstoffe, Schmiermittel, Harnsäurelösungen und dgl. denkbar, die zu reinigen sind.

Der Filter kann in Personenkraftwägen, Lastkraftwägen, Baumaschinen, Wasserfahrzeugen, Schienenfahrzeugen, Luftfahrzeugen sowie allgemein in der Klimatechnik, insbesondere in Heiz-Klimageräten, in Haushaltsgeräten, in Brennstoffzellen oder in der Gebäudetechnik Anwendung finden. Diese Fahrzeuge können elektrisch und/oder mittels Kraftstoff (insbesondere Benzin, Diesel oder Erdgas) betrieben werden. Im Hinblick auf die Gebäudetechnik kommen insbesondere stationäre Anlagen zur Behandlung von Luft, beispielsweise für die stationäre Stromerzeugung in Betracht.

Weitere mögliche Implementierungen der Erfindung umfassen auch nicht explizit genannte Kombinationen von zuvor oder im Folgenden bezüglich der Ausführungsbeispiele beschriebenen Merkmale oder Verfahrensschritte. Dabei wird der Fachmann auch Einzelaspekte als Verbesserungen oder Ergänzungen zu der jeweiligen Grundform der Sensoreinrichtung oder des Filters hinzufügen.

Weitere Ausgestaltungen der Erfindung sind Gegenstand der Unteransprüche sowie der im Folgenden beschriebenen Ausführungsbeispiele. Im Weiteren wird die Erfindung anhand von Ausführungsbeispielen unter Bezugnahme auf die beigelegten Figuren näher erläutert.

### Kurze Beschreibung der Zeichnung

Es zeigt dabei:
- Fig. 1 - 3:: schematische Schnitt- und Perspektivdarstellungen einer ersten Ausführungsform einer Sensoreinrichtung;
- Fig. 4 - 7:: schematische Schnitt- und Perspektivdarstellungen einer Ausführungsform einer Filteranordnung mit einer zweiten Ausführungsform einer Sensoreinrichtung;
- Fig. 8 und 9:: schematische Schnitt- und Perspektivdarstellungen einer dritten Ausführungsform einer Sensoreinrichtung; und
- Fig. 10: eine schematische Teil-Schnittdarstellung einer vierten Ausführungsform einer Sensoreinrichtung.

### Ausführungsform(en) der Erfindung

Im Folgenden werden Ausführungsformen von Filteranordnungen und Sensoreinrichtungen anhand von Vorfiltern für Dieselkraftstoff beschrieben. Dabei ist insbesondere ein Wassersensor als Sensorelement angegeben. An die Ausführungsbeispiele angelehnt können andere Filteranordnungen mit abweichenden Betriebsmitteln, Filtermedien oder Sensoren eingesetzt werden.

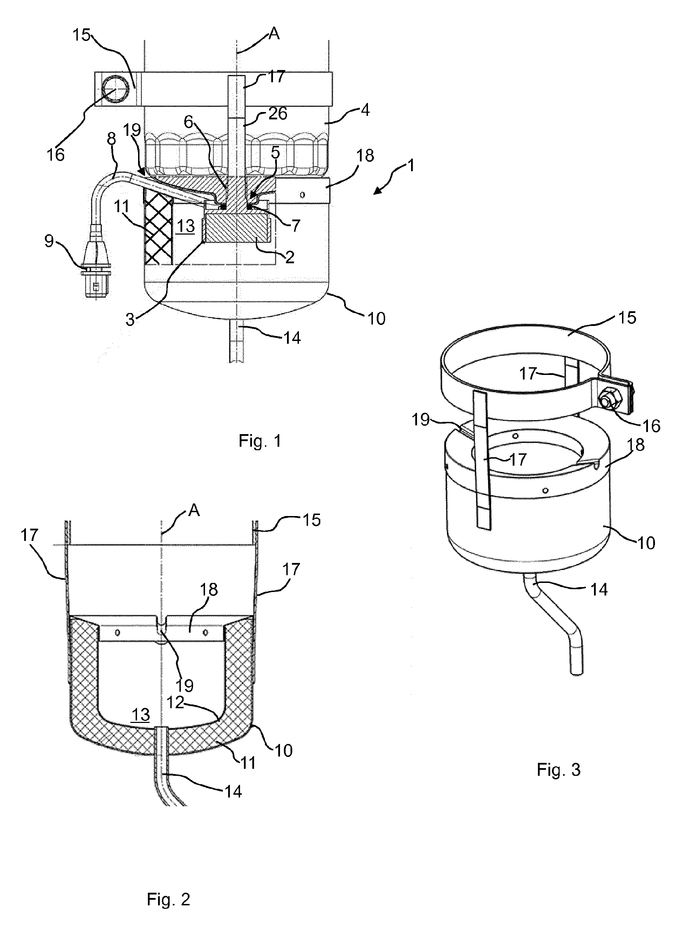

Fig. 1 - 3 zeigen schematische Schnitt- und Perspektivdarstellungen einer ersten Ausführungsform einer Sensoreinrichtung 1. Die Fig. 1 zeigt dabei eine Schnittansicht durch eine Sensoreinrichtung 1, welche an einen als Wechselfilter ausgebildeten Fluidfilter 4 gekoppelt ist. Die Fig. 2 zeigt einen Schnitt durch einen als Brandschutzgehäuse wirkenden Abschnitt an der Sensoreinrichtung, und Fig. 3 eine perspektivische Darstellung desselben.

Im oberen Bereich der Fig. 1 ist schematisch ein Wechselfilter 4 dargestellt. Der Wechselfilter 4 ist zylindrisch ausgeführt und hat in der Orientierung in Fig. 1, welche die typische Einbausituation widerspiegelt, unten eine Öffnung 5 zum Anschluss eines Sensorelements 2. Der Wechselfilter 4 hat dabei beispielsweise einen Filtertopf aus einem Metallwerkstoff. Das Wechselfilter4 dient dabei dem Filtern von Kraftstoff und dem Abscheiden von Wasser aus dem Kraftstoff.

Beim Betrieb des Wechselfilter4 sammelt sich im unteren Bereich abgeschiedenes Wasser als Flüssigkeit. Um den Füllstand bzw. das Vorliegen von Wasser zu erkennen, ist eine Sensoreinrichtung 2 vorgesehen. Die Sensoreinrichtung 2 ist in einem meist aus Kunststoff gefertigten Sensorgehäuse 3 untergebracht und reicht mit einem Messfühler 26 in den Innenraum des Filtertopfes des Elementes 4.

Das Sensorelement 2 ist fluiddicht mit dem Gehäuse des Wechselfilters 4 verbunden. Dazu ist ein Dichtring 7 vorgesehen, der insbesondere eine axiale Dichtwirkung entlang der Mittelachse A der sich ergebenden Filteranordnung mit der Sensoreinrichtung 1 erstreckt. Alternativ kann auch eine Dichtung in radialer Richtung oder schräg mit radialem und axialem Anteil erfolgen. Zum Verbinden des Sensorelementes 2 mit dem Wechselfilter4 kann ein Gewindestück 6 vorgesehen sein oder andere Verbindungsmittel, die hier nicht weiter dargestellt sind. Beim Betrieb des Filters bzw. Wechselfilters 4 entstehen Drücke von bis zu 8 bar, d.h. der Abschluss des Wechselfilters 4 an der Öffnung 5 mit Hilfe des Sensorelements 2 widersteht diesen Drücken.

Das Sensorelement 3 liefert über ein Anschlusskabel 8, das hier seitlich nach außen reicht, und einem daran angeschlossenen Steckverbinder 9 Sensorsignale an eine Auswerteeinrichtung. Dies kann beispielsweise eine Fahrzeugelektronik oder Auswerteschaltung sein. Es ist auch denkbar, dass die Sensoreinrichtung 1 Signale an einen Fahrzeugbus, wie beispielsweise einen CAN-Bus, liefert.

Das Sensorelement 2 ist nach außen von einem Brandschutzgehäuse 10 umgeben, das hülsen- oder topfförmig ausgestaltet ist. Das Gehäuse ist in Fig. 3 perspektivisch dargestellt und umfasst einen unteren Brandschutzgehäuseteil 10, der becher- oder topfförmig ausgestaltet ist. Ein oberes Übergangsstück 18 ist mit einem Durchgang 19 für das Anschlusskabel 8 versehen und schmiegt sich an die Kontur des unteren Bereichs des Wechselfilters 4 an. Dies erkennt man insbesondere in Fig. 1.

Das Brandschutzgehäuse 10 ist mit einer Schelle 15 und einem bzw. zwei Haltestreifen 17 an das Wechselfilter 4 befestigt. Dazu wird die als Schlauchschelle ausgestaltete Schelle 15 mit einem Schraubmittel 16 befestigt. An dem Brandschutzgehäusetopf 10 ist unterseitig ein Ablauf oder Ablaufrohr 14 vorgesehen, durch den im Inneren des Brandschutzgehäuses 10 vorliegende Flüssigkeit ablaufen kann. Der Ablauf 14 ist dabei gekröpft seitlich weggebogen gestaltet. Dadurch können potenziell Flammen kaum oder nicht durch die gekröpfte Rohrleitung bzw. den Abfluss 14 eindringen.

In Fig. 1 ist gestrichelt eine Sicht in das Brandschutzgehäuse 10 angedeutet. Die Fig. 2 zeigt einen Längsschnitt durch das Brandschutzgehäuse 10, in dem das Sensorelement 2 aufgenommen ist. Das Brandschutzgehäuse 10 ist dabei mit einem Brandschutzmaterial 11 versehen, das in der Art eines Bechers das Sensorelement 2 umschließt. Oberseitig grenzt das Brandschutzmaterial 11 an die Unterseite des Wechselfilters 4. Im eingebauten Zustand erkennt man in Fig. 1, dass ein Hohlraum 13 zwischen dem Sensorgehäuse 3 und der Innenseite 12 des Brandschutzgehäuses 10 vorliegt. Der Hohlraum 13 oder Spalt dient im Brandfall als weitere Isolation.

Das Brandschutzmaterial 11, welches schraffiert dargestellt ist, umfasst beispielsweise ein Mineral, eine keramische Vergussmasse oder Glasfasermaterial, welches hohen Temperaturen und Beflammungen widersteht. Man kann auch von einer Flammschutzumhüllung des Sensorelementes 2 sprechen.

Alternativ oder zusätzlich kann das Brandschutzmaterial 11 einen Brandschutzschaum umfassen, der bei Wärmeeinwirkung aufquillt bzw. expandiert und damit von außen eintretende Flammen bzw. Hitze von dem Sensorelement bzw. der Dichtung 7 fernhält. Die Sensoreinrichtung 1 ist insbesondere derart mit dem Brandschutzgehäuse 10 ummantelt, dass eine Flammschutzprüfung nach den oben genannten Vorschriften bestanden wird. D.h., aus dem Inneren des Wechselfilters 4 tritt auch nach 30-minütiger Flammeneinwirkung bzw. einer Temperaturerhöhung auf über 850°C keine Filterfluid aus.

Bei den Ausführungsformen sind insbesondere Materialien, die einen Schmelzpunkt von kleiner als etwa 930°C haben, mit dem Brandschutzmaterial ummantelt. Die Elemente der Sensoreinrichtung 1, welche zum Befestigen des Brandschutzmaterials 11 dienen, sind beispielsweise aus einem Metallwerkstoff gefertigt. Dies sind insbesondere die Schelle 15, der Haltestreifen 19, der Brandschutzgehäusetopf 10 sowie das Ablassrohr 14.

Die vorgeschlagene Sensoreinrichtung 1 ermöglicht insbesondere einen Betrieb entsprechend ausgestatteter Vorfilteranordnungen für Dieselmotoren in Marineanwendungen in Wasserfahrzeugen. Der eigentliche Sensor 2 kann dabei mit einem Sensorgehäuse 3 aus einem Polyamidmaterial gefertigt sein.

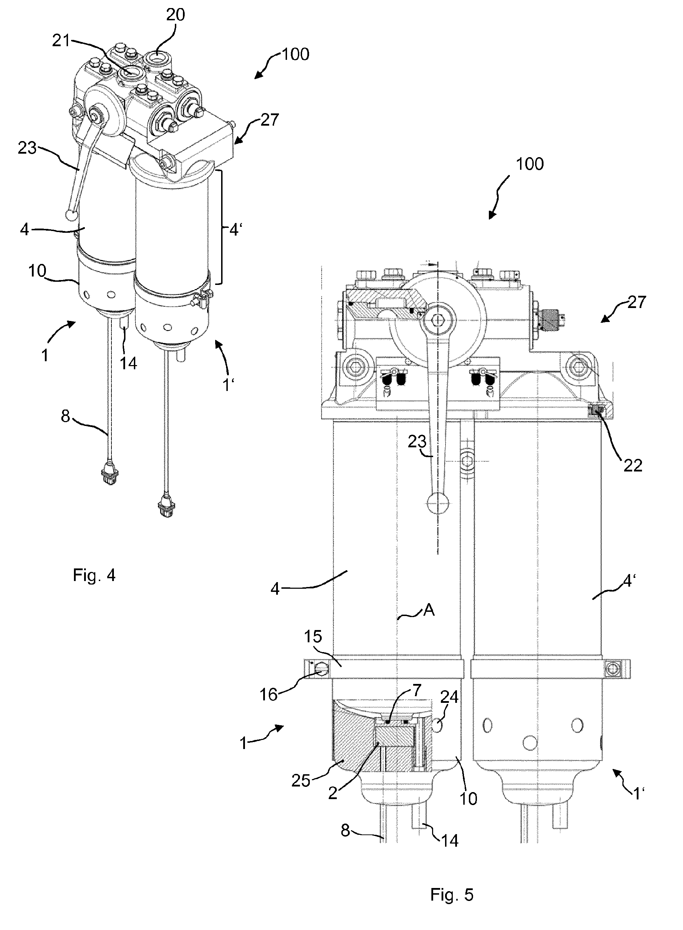

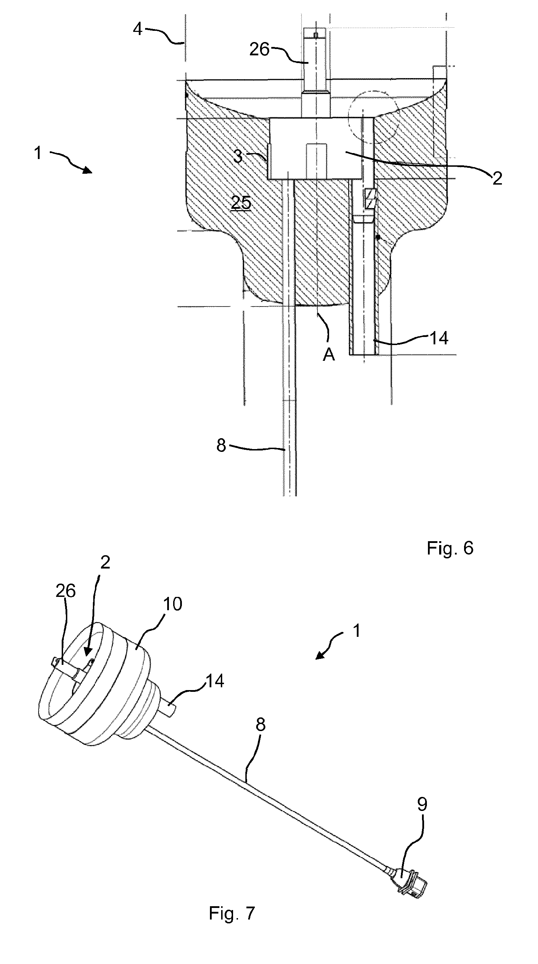

Fig. 4 bis 7 zeigen schematische Schnitt- und Perspektivdarstellungen einer Ausführungsform einer Filteranordnung und Elemente darin. Dabei zeigt Fig. 4 eine perspektivische Ansicht der Filteranordnung, Fig. 5 eine Schnittansicht der Filteranordnung, Fig. 6 eine Schnittanordnung der eingesetzten Sensoreinrichtung und Fig. 7 eine perspektivische Ansicht der Ausführungsform der Sensoreinrichtung. Die in den Fig. 1 -3 beschriebenen Elemente der Sensoreinrichtung können gleich oder ähnlich auch hier eingesetzt werden.

Die Fig. 4 zeigt eine perspektivische Darstellung einer Vorfilteranordnung 100, insbesondere zum Einsatz für Dieselmotoren in oder auf Wasserfahrzeugen. Die Filteranordnung 100 ist als Doppelfilter mit zwei Wechselfiltern 4, 4' ausgestattet. Die Filteranordnung hat dabei einen Filterkopf 27, der insbesondere einen Einlass 20 für zu reinigendes Fluid, wie Dieselkraftstoff, und einen Auslass für gereinigten Kraftstoff 21 umfasst. An dem Filterkopf 27 sind verschiedene Armaturen und Anschlussstücke für die Wechselfiltere 4, 4' vorgesehen, auf die im Folgenden nicht weiter eingegangen wird.

Ein Hebel 23 ermöglicht das Zu- und Abschalten der beiden Wechselfilter 4, 4' unabhängig voneinander. Beispielsweise kann bei der Hebelstellung 23 nach links (vgl. Fig. 5) der linke Wechselfilter 4 vom Kraftstoff und Filterkreislauf abgekoppelt und ausgetauscht werden. Durch die zwei unabhängig voneinander betreibbaren Wechselfilter 4, 4' kann während des Betriebs des Filters bzw. der Filteranordnung 100 ein Austausch einzelner Wechselfilter erfolgen, ohne den Betrieb zu unterbrechen. Es sind jedoch auch Einzelanordnungen mit oder ohne Absperrhahn denkbar.

Die Wechselfilter sind mit Hilfe von Dichtringen 22, wie in Fig. 5 angedeutet ist, an den Filterkopf 27 fluiddicht verbunden. Im Folgenden wird im Wesentlichen auf die linke mit dem Bezugszeichen 4 versehene Kombination aus Wechselfilter4 und Sensoreinrichtung 1 eingegangen.

In Fig. 5 erkennt man ein von einem Brandschutzschaum 25 (schraffiert dargestelt) umgebenes, insbesondere in dem Brandschutzschaum 25 eingebettet aufgenommenes Sensorelement 2, das über eine Dichtung 7 an den Wechselfilter 4 gekoppelt ist. Der Brandschutzschaum 25 ist bevorzugt als eigenstabiler Formkörper ausgebildet. Der Brandschutzschaum ist radial außen von einem als Rohrzylinder ausgebildeten Brandschutzgehäuse 10, beispielsweise aus Stahlblech, insbesondere aus rostfreiem Edelstahlblech, umgeben. Das Brandschutzgehäuse 10 ist mit dem Wechselfilter 4, 4` verbunden und leicht (ca. 0,3-2 mm) vom Brandschutzschaum beabstandet, so dass dieser innerhalb des Brandschutzgehäuses 10 drehbar angeordnet werden kann. Die Drehbarkeit ist zur Montage der Brandschutzschaum-Sensorelement-Einheit wünschenswert und für das regelmäßige Ablassen des im Wechselfilter 4 abgeschiedenen Wassers erforderlich, welches durch Anlösen des Sensorelements erfolgt. Das Brandschutzgehäuse 10 ist über eine Schelle 15 und Schrauben 16 an den Wechselfilter 4 befestigt. Das Brandschutzmaterial 25 ist dabei ein Polyurethanschaum, der beispielsweise mit halogenfreien Brandschutzadditiven wie Clariant Exolit OP 560 oder Lanxess Uniplex FRP versetzt ist und intumeszierende Eigenschaften hat. Bei einer Beflammung von außen dehnt sich der Brandschutzschaum aus und verformt sich durch die im Brandschutzgehäuse 10 vorgesehenen Öffnungen 24 hinaus. Aufgrund der Volumenzunahme schützt der expandierte Brandschutzschaum das innenliegende Sensorelement 2 über einen gewissen Zeitraum vor Zerstörung bzw. Verbrennen. Bevor eine Flamme das Sensorelement 2 und die Dichtung 7 erreicht, brennt zunächst der expandierte Brandschutzschaum 25 ab.

In Fig. 5 ist ein aufgebrochenes Fenster gestrichpunktet unten links an der Sensoreinrichtung 1 dargestellt. Das Sensorelement 2 umfasst ein Anschlusskabel 8, das exzentrisch zur Mittelachse A der Filteranordnung 100 und des Sensorelements 2 nach unten durch den Brandschutzschaum 25 geführt ist. Ferner ist ein Wasser- bzw. Flüssigkeitsablasskanal 14 ebenfalls exzentrisch zur Mittelachse A geführt.

In Fig. 6 ist ein Ausschnitt der linken Sensor- und Filtereinrichtung 1 im Schnitt dargestellt. Das Sensorelement 2 ist mit einem Messfühler 26, der in den Innenraum des Wechselfilters 4 reicht, ausgestattet. Die Verbindung zwischen dem Innenraum des Wechselfilters 4 und dem Sensorelement 2 ist fluiddicht. Seitlich vom Sensorelement 2 kann ein Wasserablasskanal 14 durch den Bereich des Brandschutzmaterials 25 hindurch nach außen geführt sein. Das Brandschutzmaterial 25 verfängt sich beispielsweise beim Expandieren an den in Fig. 5 als 24 bezeichneten Öffnungen des Brandschutzgehäuses 10. Ferner können weitere Mittel, wie beispielsweise raue Oberflächen, Auskragungen oder abstehende Blechbereiche unterhalb der Öffnung 24 vorgesehen sein, die ein Herabfallen oder Abtropfen von ausgetretenem Brandschutzschaum behindert.

Die Fig. 7 zeigt eine perspektivische Darstellung der Sensoreinrichtung 1, wie sie im Ausführungsbeispiel für die Filteranordnung nach den Fig. 4 und 5 eingesetzt ist. Das topf- oder becherförmige, beispielsweise aus Blech gefertigte Brandschutzgehäuse 10 ist vorzugsweise mit dem Brandschutzschaum 25 verfüllt. Aus dem Bereich des Brandschutzschaumes austretend sieht man in Fig. 7 das Anschlusskabel 8 mit einem Steckverbinder 9 sowie den Wasserablass 14.

Bei bestimmungsgemäßem Gebrauch weist die Mittel- oder Symmetrieachse A der Filteranordnung 100 und/oder der Sensoreinrichtung 1 vertikal nach unten, also entlang der Erdbeschleunigung. Dadurch kann Wasser nach unten durch den Ablass 14 austreten. Aufgrund des Flamm- oder Brandschutzmaterials um die Sensoreinrichtung bzw. das meist aus Kunststoffmaterialien gebaute Sensorelement 3 kann über einen gewissen Zeitraum bei Auftreten eines Brandes in der Nähe der Filteranordnung 100 ein zuverlässiger Betrieb noch gewährleistet werden.

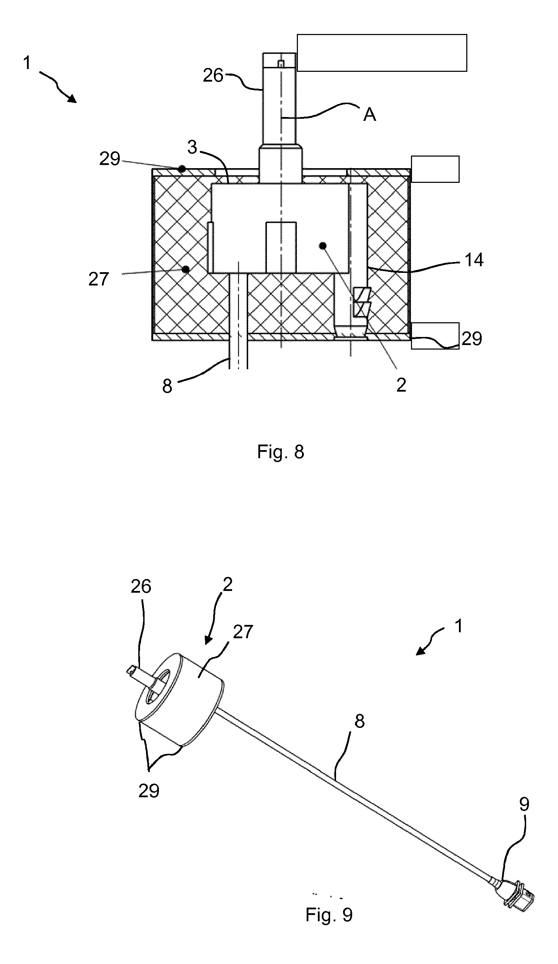

Die Fig. 7 und 8 zeigen eine dritte Ausführungsform einer Sensoreinrichtung 1. Fig. 8 ist ein Längsschnitt durch die Sensoreinrichtung ähnlich Fig. 6, und Fig. 9 ist eine perspektivische Darstellung der Sensoreinrichtung. Es sind im Wesentlichen die gleichen Elemente wie in der ersten und zweiten Ausführungsform vorhanden und mit den gleichen Bezugszeichen versehen, worauf hier nicht mehr explizit eingegangen wird. Die Brandschutzummantelung des Sensorelements 2 erfolgt durch eine keramische Vergussmasse 27, wie eine feuerfeste Gießmasse auf Basis von Korund mit Kalziumaluminatbindung. Das Brandschutzmaterial 27 hat zum Beispiel einen Gewichtsanteil zwischen 95% und 99% und einen Kalziumoxidanteil zwischen 1 % und 5%. Das keramische Brandschutzmaterial 27 ist zwischen zwei Deckplatten 29 aus einem Blähgraphit vorgesehen.

Die Sensoranordnung 1 hat kein Brandschutzgehäuse, sondern das keramische Brandschutzmaterial 27 hält stabil an dem Sensorelement 3, und ist nach oben und unten mit jeweils einer Deckplatte 29 beklebt. Die Deckplatten haben zum Beispiel eine Dicke zwischen 1 und 3 mm und die zylinderförmige Brandschutzkeramik hat eine Höhe zwischen 40 und 60 mm. Das Anschlusskabel 8 und der Steckverbinder 9 genügen zum Beispiel der DIN 5510, so dass ein Einsatz der Sensoreinrichtung 1 und der daran angekoppelten (hier nicht dargestellten) Filtereinrichtung in besonders in Anwendungen mit speziellen Sicherheitsanforderungen eingesetzt werden kann.

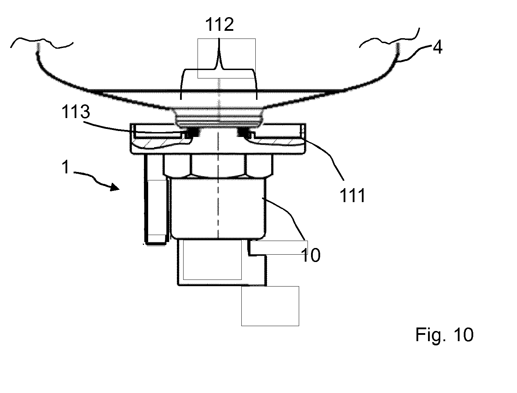

In der in Figur 10 gezeigten Ausführungsform einer Sensoranordnung 1 ist das Brandschutzmaterial ein rostfreies Edelstahlmaterial, welches das Gehäuse 10 des Sensorelementes bildet und dieses vollständig umgibt. Auf das Gehäuse 10 einwirkende Wärme kann teilweise über das Sensorelement an die im Inneren des Filters 4 und des Sensors befindliche Betriebsflüssigkeit abgeführt werden, was den Aufwärmvorgang im Brandfalle verzögert. Das Gehäuse 10 ist dabei bevorzugt im an den Filter angrenzenden Bereich mit einer ringscheibenförmigen Auskragung 111 versehen, die radial deutlich (d.h. mindestens 5 mm und bevorzugt mindestens 10 mm) über den Anschlussbereich 112 des Sensorelementes mit Anschlussgewinde (nicht gezeigt) und Dichtung 113 zur Abdichtung gegenüber dem Wechselfilter 4 hinausragt. Auf diese Weise können von räumlich unten an die Sensoranordnung heranreichende Flammen abgelenkt werden, so dass die Gefahr eines direkten Kontaktes des Anschlussbereiches 113 mit Flammen deutlich verringert wird.

Entgegen üblichen aus Kunststoff gefertigten Sensoren ergibt sich durch die Ummantelung mit einer Brandschutzschicht oder einem Brandschutzmaterial eine erweiterte Einsatzmöglichkeit. Insbesondere in Anwendungen, bei denen besondere Brandschutzanforderungen bestehen, wie beispielsweise im Marinebereich auf Schiffen, kann der vorgeschlagene PreLine- oder Vorfilter für Kraftstoffe eingesetzt werden. Die Filteranordnung, und insbesondere die Sensoranordnung, entspricht dabei vorzugsweise den in der DIN EN ISO 7840 und/oder ISO 19 921 dargelegten Anforderungen an die Feuerwiderstandsfähigkeit. Es wird insbesondere eine Fluiddichtigkeit am Übergang zwischen dem Sensorelement 2 und dem Wechselfilter 4 über wenigstens 30 min bei einer Flammtemperatur von 700 - 900°C erreicht.

Obwohl die vorliegende Erfindung anhand bevorzugter Ausführungsbeispiele näher erläutert wurde, ist sie nicht darauf beschränkt, sondern vielfältig modifizierbar. "Ein" schließt vorliegend keine Vielzahl aus.

## Patentansprüche

1. Sensoreinrichtung (1) für eine Filteranordnung (100), mit mindestens einem Sensorelement (2) zum Erfassen eines Betriebsparameters für die Filteranordnung (100), insbesondere zum Detektieren von Wasser, und mit einem Brandschutzmaterial (11, 25), welches das Sensorelement (2) zumindest teilweise umgibt.

2. Sensoreinrichtung (1) nach Anspruch 1, wobei das Brandschutzmaterial (25) ein intumeszierendes Material umfasst.

3. Sensoreinrichtung (1) nach Anspruch 1 oder 2, wobei das Brandschutzmaterial (11) ein flammbeständiges Fasermaterial umfasst.

4. Sensoreinrichtung (1) nach einem der Ansprüche 1 bis 3, ferner mit einem das Sensorelement (2) umschließenden Brandschutzgehäuse (10) zur Aufnahme des Brandschutzmaterials (11, 25) um das Sensorelement (2) herum.

5. Sensoreinrichtung (1) nach einem der Ansprüche 1 bis 4, wobei zwischen dem Brandschutzmaterial (11) und dem Sensorelement (2) ein Hohlraum (13) vorgesehen ist.

6. Sensoreinrichtung (1) nach einem der Ansprüche 1 bis 5, wobei das Sensorelement (2) mindestens eine Anschlussleitung (8) zum Übermitteln von Sensorsignalen aufweist, wobei die Anschlussleitung (8) wenigstens teilweise durch das Brandschutzmaterial (11, 25) nach außen geführt ist.

7. Sensoreinrichtung (1) nach einem der Ansprüche 1 bis 6, wobei das Brandschutzmaterial (11, 25) derart ausgestaltet ist, dass das Sensorelement (2) einer Beflammung der Sensoreinrichtung (1) über mindestens dreißig Minuten bei mindestens 800 °C widersteht.

8. Filteranordnung (100) mit einem Filterelement (4) zum Filtern eines Fluids und einer Sensoreinrichtung (1) nach einem der Ansprüche 1 bis 7, wobei das Sensorelement (2), insbesondere mit Hilfe einer Dichtung (7), fluiddicht an das Filterelement (4) angebracht ist.

9. Filteranordnung nach Anspruch 8, ferner mit einer Halteeinrichtung (15, 17) für das Filterelement (4), das Brandschutzgehäuse (10) und/oder eines expandierenden Brandschutzmaterials (11, 25).

10. Verfahren zum Herstellen einer Sensoreinrichtung (1) nach einem der Ansprüche 1 bis 7, wobei ein Sensorelement (1) mit einem fluiden Brandschutzmaterial (25) wenigstens teilweise ummantelt wird, und das Brandschutzmaterial (25) in einem abgebundenen verfestigten Zustand intumeszierend ist.

11. Verfahren nach Anspruch 10, wobei das fluide Brandschutzmaterial (25) ein Polyurethan-Werkstoff ist, der in flüssiger Form in einer Gießschale eindosiert wird, in welcher bereits das Sensorelement positioniert ist, wobei der Polyurethanwerkstoff beim Eindosieren und/oder beim Aufschäumen das Sensorelement umschließt und anschließend aushärtet, so dass das Sensorelement (1) formschlüssig und insbesondere Drehmomentfest ummantelt ist.
